(19) Europäisches Patentamt
European Patent Office
Office européen des brevets

(11) EP 1 023 033 B1

(12) **FASCICULE DE BREVET EUROPEEN**

(45) Date de publication et mention de la délivrance du brevet:
**29.10.2003 Bulletin 2003/44**

(21) Numéro de dépôt: **98912575.2**

(22) Date de dépôt: **02.03.1998**

(51) Int Cl.[7]: **A61K 7/00**

(86) Numéro de dépôt international:
**PCT/FR98/00403**

(87) Numéro de publication internationale:
**WO 98/038969 (11.09.1998 Gazette 1998/36)**

(54) **COMPOSITION DE COIFFAGE REMODELABLE**

ZUSAMMENSETZUNG ZUR GESTALTUNG VERÄNDERBARER FRISUREN

HAIRSTYLING COMPOSITION CAPABLE OF BEING REMODELLED

(84) Etats contractants désignés:
**AT BE CH DE DK ES FI FR GB GR IE IT LI LU MC NL PT SE**

(30) Priorité: **04.03.1997 FR 9702558**

(43) Date de publication de la demande:
**02.08.2000 Bulletin 2000/31**

(73) Titulaire: **L'OREAL**
**75008 Paris (FR)**

(72) Inventeurs:
- **ROLLAT, Isabelle**
  **F-92100 Boulogne (FR)**
- **DUPUIS, Christine**
  **F-75018 Paris (FR)**
- **SAMAIN, Henri**
  **F-91570 Bièvres (FR)**

(74) Mandataire: **Miszputen, Laurent et al**
**L'OREAL - D.I.P.I.**
**25-29 Quai Aulagnier**
**92600 Asnières (FR)**

(56) Documents cités:
**EP-A- 0 320 218**
**EP-A- 0 758 545**
**EP-A- 0 758 546**
**EP-A- 0 764 437**
**WO-A-91/15186**
**WO-A-92/16179**
**WO-A-97/09030**
**WO-A-97/29734**
**WO-A-97/33558**
**FR-A- 2 737 660**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une composition de coiffage remodelable, permettant de modifier la coiffure tout en préservant sa tenue, sans avoir à remouiller les cheveux ou les chauffer de manière excessive. Elle concerne également un dispositif particulier approprié pour la distribution de la composition selon l'invention. Elle concerne enfin un procédé de traitement des fibres kératiniques, en particulier des cheveux, pour lequel on applique la composition selon l'invention sur lesdites fibres.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît également les gels ou les mousses de coiffage qui sont généralement appliqués sur les cheveux mouillés avant de faire un brushing ou une mise en plis. A la différence des laques aérosols classiques, ces compositions présentent l'inconvénient de ne pas permettre la fixation des cheveux dans une forme déjà réalisée. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage.

Les compositions de l'état de la technique présentent toutes le même inconvénient de ne pas permettre une modification de la coiffure dans une forme souhaitée autre que celle initialement formée, à moins de ne recommencer les opérations de coiffage et de fixation. En outre, sous une contrainte quelconque, la coiffure a tendance à prendre un pli permanent non souhaité, qu'il n'est pas possible de modifier facilement.

On connaît de la demande de brevet EP 524 346, une composition permettant d'obtenir un coiffage modifiable après fixation. Toutefois, pour pouvoir modifier la coiffure, il est nécessaire de la chauffer à une température supérieure à la température de transition vitreuse (Tg) du matériau fixant, laquelle peut aller jusqu'à 120 °C.

La Demanderesse a maintenant trouvé qu'en sélectionnant certains polymères appropriés, seuls ou en combinaison avec certains additifs dans un véhicule approprié cosmétiquement acceptable, il était possible d'obtenir un coiffage remodelable, à savoir d'obtenir un matériau coiffant spécifique permettant de modifier la coiffure tout en préservant sa tenue, sans avoir à remouiller les cheveux ou les chauffer de manière excessive

Les matériaux coiffants appropriés pour obtenir cet effet de coiffage remodelable selon l'invention doivent d'une part avoir, après application sur le support et séchage, une température de transition vitreuse (Tg) inférieure à +10 °C.

D'autre part, ils doivent présenter, après application sur le support et séchage, un profil de décollement défini par au moins :

(a) une force maximale de décollement $F_{max}$ > 1 Newton et
(b) lorsque la température de transition vitreuse (Tg) est inférieure à -15°C, par une énergie de séparation $E_{s(M/V)}$ du matériau mis en contact avec une surface en verre, inférieure à 300 µJ.

Selon la présente invention, on entend par « Fmax » : la force maximale de traction, mesurée à l'aide d'un extensomètre, nécessaire pour décoller les surfaces de 38 $mm^2$, respectives de deux supports (A) et (B), rigides, inertes, non-absorbants, placés en regard l'un de l'autre ; lesdites surfaces étant enduites préalablement par ladite composition, à raison de 53/c $\mu g/mm^2$, c étant la concentration en matière sèche dans la composition (en gramme par gramme de composition) c'est-à-dire le rapport de la masse en matière sèche dans la composition sur la masse totale de la composition, et séchées pendant 24 heures à 22°C sous une humidité relative de 50%, puis soumises pendant 20 secondes à une compression de 3 Newtons et enfin soumises pendant 30 secondes à une traction de vitesse 20 mm/minute.

Selon la présente invention, on entend par « $E_{s(M/V)}$ » : l'énergie fournie par l'extensomètre pour effectuer la « séparation » des surfaces respectives de 38 $mm^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) définis ci-dessus et dont la surface est enduite de la composition, à raison de 53/c $\mu g/mm^2$, c étant la concentration en matière sèche dans la composition (en gramme par gramme de composition), c'est-à-dire le rapport de la masse en matière sèche dans la composition sur la masse totale de la composition, et séchée pendant 24 heures à 22°C sous une humidité relative de 50% ; les deux surfaces desdits supports(C) et (D) étant soumises ensuite pendant 20 secondes à une compression de 3 Newtons et enfin soumises

pendant 30 secondes à une traction de vitesse 20 mm/minute.

Cette énergie fournie par l'extensomètre est le travail calculé au moyen de la formule suivante :

$$\int_{X_{s1}+0,05}^{X_{s2}} F(x)dx$$

où F(x) est la force nécessaire pour produire un déplacement (x);

$x_{s1}$ est le déplacement (exprimé en millimètres) produit par la force de traction maximale ;

$X_{S2}$ est le déplacement (exprimé en millimètres) produit par la force de traction permettant la séparation totale des deux surfaces des supports (C) et (D) définis ci-dessus.

La présente invention concerne donc une composition de coiffage remodelable comprenant dans un véhicule cosmétique approprié pour les fibres kératiniques et plus particulièrement pour les cheveux, au moins un polymère choisi de telle sorte qu'elle conduise, après application sur les fibres et séchage, à un matériau coiffant tel que défini ci-dessus.

Par température de transition vitreuse (Tg), on entend selon la présente invention la Tg du matériau coiffant obtenu après application sur le support et séchage de la composition, à l'exception des matériaux volatiles éventuellement présents. La température de transition vitreuse est déterminée par D.S.C. (méthode calorimétrique).

Par matériau coiffant, on entendra dans toute la description tout matériau permettant d'obtenir l'effet de coiffage remodelable recherché.

De manière préférentielle, le matériau coiffant est essentiellement constitué par au moins un polymère fixant, seul ou en combinaison avec des additifs cosmétiques usuels, par exemple des plastifiants, ou des agents neutralisants.

Selon l'invention, on peut utiliser tout polymère fixant connu en soi, à la condition que la Tg du matériau coiffant obtenu soit inférieure à +10 °C et vérifie les caractéristiques d'adhésivité telles qu'indiquées ci-dessus.

Dans le cas où le matériau coiffant est constitué d'un mélange de polymères et d'autres constituants, il n'est pas nécessaire que la Tg de chacun des constituants polymériques soit inférieure à +10°C car il existe de nombreuses possibilités pour ajuster la Tg par mélanges (mélanges de polymères, plastifiants).

On peut utiliser en particulier des polymères fixants choisis parmi les polymères anioniques, cationiques, amphotères, non ioniques et leurs mélanges. Le polymère fixant peut en outre être halogéné, en particulier fluoré.

Les polymères fixants peuvent être utilisés sous forme solubilisée ou encore sous forme de dispersions de particules solides de polymère (latex ou pseudo-latex).

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000.

Des polymères fixants anioniques particulièrement préférés selon l'invention sont les polyesters sulfonés comprenant des unités répétitives pouvant être représentés par la formule générale suivante:

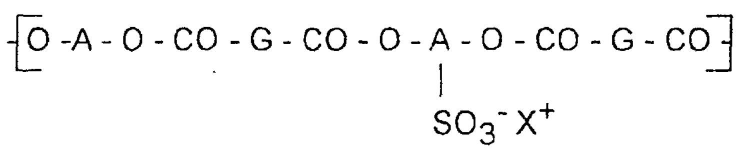

dans laquelle A et G représentent des radicaux divalents et X représente un métal alcalin, en particulier sodium ou potassium. Parmi les polyesters sulfonés préférés selon l'invention, A représente un radical arylène, en particulier phénylène et G représente un radical alkylène linéaire ou ramifié, éventuellement interrompu par un ou plusieurs atomes d'oxygène, ou cycloalkylène. Lorsque G représente un radical alkylène linéaire ou ramifié, éventuellement interrompu par un ou plusieurs atomes d'oxygène, le radical - O - G - O - est de préférence un reste de (poly)alkylène glycol comprenant 1 à 20 unités alkylène glycol. Le radical alkylène est de préférence selon l'invention un radical alkylène inférieur linéaire ou ramifié en $C_2$-$C_4$, plus préférentiellement un radical éthylène. De tels polymères sont notamment décrits dans les brevets US 3 546 008, US 4 340 519, US 3 734 874, US 3 779 993, US 4 233 196 et US

5 386 003 et dans la demande de brevet WO 95/32997. Parmi ces polymères, on préférera ceux commercialisés sous les dénominations AQ 1045, AQ 1350 et AQ 14000 par la société EASTMAN CHEMICAL, plus particulièrement l'AQ 1350.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire. De tels polymères amphotères sont notamment décrits dans les brevets et demandes de brevet US 3 836 537 et FR 1 400 366.

Les polymères fixants anioniques ou amphotères peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique.

Les polymères fixants non ioniques utiles selon la présente invention sont notamment des polyuréthannes.

Les caractéristiques relatives au profil de décollement du matériau coiffant de l'invention peuvent être mesurées et calculées selon les modes opératoires suivants :

Mode opératoire relatif à la mesure du Fmax

On détermine la force $F_{max}$ de traction maximale nécessaire pour séparer deux surfaces de deux supports, rigides, inertes et non-absorbants et placés en regard l'un de l'autre enduites par le matériau coiffant à évaluer à l'aide d'un extensomètre, par exemple un appareil du type LLOYD modèle LR5K.

Les supports solides, rigides, inertes et non absorbants peuvent être choisis parmi ceux constitués de polyéthylène, de polypropylène, d'alliage métallique et plus préférentiellement de verre.

On utilise de préférence à titre de supports une paire de plots constitués d'un disque de verre surmontant une tige nécessaire pour l'accroche par les mors de l'extensomètre. Ledit disque est de préférence de la taille du plot et fixé à celui-ci par une colle du type ARALDITE®. La composition de coiffage à tester est répartie de façon la plus uniforme possible sur la surface de chaque disque de verre et mise à sécher de façon à ce que la surface reste plane.

On utilise des disques de surface de 38 $mm^2$. La quantité de composition déposée est de 53/c $\mu g/mm^2$, c étant la concentration en matière sèche dans la composition (en gramme par gramme de composition). Le temps de séchage est de 24 heures à 22°C sous une humidité relative de 50%.. Les tiges des deux plots sont positionnées dans les mors de l'extensomètre. Les surfaces des disques enduites sont soumises ensuite à une phase de compression de 3 Newtons pendant 20 secondes par l'extensomètre. La traction est effectuée avec une vitesse de 20mm/minute pendant 30 secondes.

On détermine le profil de décollement en mesurant Fmax correspondant à la force maximale de traction, mesurée à l'aide de l'extensomètre, nécessaire pour décoller les surfaces respectives des deux disques. On procédera de préférence selon le protocole suivant :

On prépare 6 paires de plots. On réalise pour chaque paire de plots , un test de décollement selon le mode opératoire indiqué ci-dessus pour le test 1. On sélectionne les résultats obtenus sur les 6 profils de décollement effectués en excluant pour chaque paire de plots, les cas où les matériaux coiffants se sont décollés de l'un des plots de la paire. On détermine pour chaque profil de décollement restant, le Fmax. On réalise la moyenne de ces mesures.

Mode opératoire relatif à la mesure du $E_{s(M/V)}$

On détermine l'énergie fournie par l'extensomètre pour effectuer la « séparation » des surfaces respectives de deux supports de 38 $mm^2$, rigides et inertes, non absorbants et placés en regard l'un de l'autre ; l'un desdits supports étant constitué de verre poli et sa surface ne sera pas enduite par la composition coiffante, et l'autre desdits supports étant de nature identique à celle des supports définis ci-dessus et dont la surface est enduite de la composition coiffante ; les deux surfaces desdits supports sont ensuite traitées dans les mêmes conditions que celles du premier mode opératoire décrit ci-dessus et en utilisant un extensomètre du même type que précédemment. De façon préférentielle, on procédera selon le protocole suivant

On prépare 6 paires de plots. On réalise pour chaque paire de plots , un test de décollement selon le mode opératoire indiqué ci-dessus. On sélectionne les résultats obtenus sur les 6 profils de décollement effectués en excluant

pour chaque paire de plots, les cas où les matériaux coiffants se sont décollés de l'un des plots de la paire. On détermine, pour chaque profil de décollement restant, le $E_{s(M/V)}$. On réalise la moyenne de ces mesures.

La composition selon l'invention peut comprendre d'autres constituants usuels en cosmétique, en particulier des agents conservateurs, des parfums, des filtres U.V., des actifs pour le soin des cheveux, etc. Il est entendu que l'homme du métier saura choisir ces constituants et leur quantité dans la composition selon l'invention de manière à ne pas altérer ses propriétés de coiffage remodelable.

Les compositions selon l'invention peuvent se présenter sous toute forme appropriée pour son application sur les cheveux connue de l'état de la technique, en particulier sous forme de composition vaporisable, de mousse, de gel, de lotion, etc.

Le véhicule approprié cosmétiquement acceptable est adapté au mode d'application choisi. Le véhicule est de préférence constitué par un solvant approprié, auquel peuvent être ajoutés des additifs tels que des agents gélifiants, des agents moussants, des silicones, etc.

Il est entendu que l'homme du métier saura choisir les constituants additionnels et leur quantité dans la composition selon l'invention, comme les constituants du véhicule de manière à ne pas altérer ses propriétés de coiffage remodelable. Il veillera en particulier à ce que la Tg du matériau coiffant soit inférieure à +10 °C et que les profils de décollement tel qu'indiqué ci-dessus soient respectés.

Selon un mode de réalisation de l'invention, la composition est une composition vaporisable soit au moyen d'une pompe, soit une composition aérosol sous pression, vaporisable au moyen d'une valve de distribution commandée par une tête de distribution, elle même comprenant une buse par laquelle la composition aérosol est vaporisée.

La composition vaporisable selon l'invention est une solution ou une dispersion comprenant au moins un polymère fixant selon l'invention et un solvant approprié.

De manière avantageuse, le solvant approprié est l'eau, un alcool ou un mélange hydroalcoolique. Par alcool on entend selon l'invention un alcool aliphatique en $C_1$-$C_4$, de préférence l'éthanol.

Lorsque la composition vaporisable selon l'invention est une composition aérosol, elle comprend en outre une quantité appropriée de propulseur.

Le propulseur est constitué par les gaz comprimés ou liquéfiés usuellement employés pour la préparation de compositions aérosols. On emploiera de manière préférentielle l'air, le gaz carbonique ou l'azote comprimés, ou encore un gaz soluble ou non dans la composition tel que le diméthyl éther, les hydrocarbures fluorés ou non, et leurs mélanges.

La présente invention concerne également un dispositif aérosol constitué par un récipient contenant une composition aérosol constituée par d'une part une phase liquide (ou jus) contenant au moins un matériau coiffant tel que défini ci-dessus dans un solvant approprié et d'autre part un propulseur, et un moyen de distribution de ladite composition aérosol.

La présente invention concerne également un procédé de traitement des fibres kératiniques, en particulier des cheveux, dans lequel on applique sur lesdites fibres la composition selon l'invention telle que définie ci-dessus, avant ou après la mise en forme de la coiffure.

La présente invention concerne également l'utilisation d'une composition telle que définie ci-dessus dans une ou pour la fabrication d'une, formulation cosmétique de coiffage remodelable.

La présente invention concerne également l'utilisation d'un polymère tel que défini précédemment comme matériau coiffant ou pour l'obtention d'un matériau coiffant dans une formulation de coffage remodelable.

Les exemples ci-après permettent d'illustrer l'invention sans toutefois chercher à en limiter la portée.

On réalise plusieurs compositions sous forme de lotions.

Les compositions sont réalisées avec différents polymères fixants. On mesure après application sur cheveux et séchage la Tg du matériau coiffant obtenu (exprimées en °C).

L'effet de coiffage remodelable est évalué sur têtes pour les compositions concentrées à 4,5% (poids/poids) en matière sèche dans un solvant adéquat.

On réalise les tests de décollement pour les différentes compositions.

Les résultats sont reportés sur les Tableaux ci-dessous.

Exemple 1 : importance de la Tg

| Polymère | Tg (°C) | Coiffage remodelable |
|---|---|---|
| AQ 1350 | **0** | **oui** |
| HYSTRETCH V43 | **- 43** | **oui** |

(suite)

| Polymère | Tg (°C) | Coiffage remodelable |
|---|---|---|
| AMERHOLD DR 25 non plastifié (ne faisant pas partie de l'invention) | **+24** | **non** |
| Néocryl BT 67 (ne faisant pas partie de l'invention) | **+14** | **non** |

Exemple 2 : importance du $F_{max}$

| Polymère | Tg (°C) | $F_{max}$ (Newtons) | Coiffage remodelable |
|---|---|---|---|
| AQ 1350 | **0** | **23** | **oui** |
| HYSTRETCH V43 | **- 43** | **3,5** | **oui** |
| KRATON G 1701 (ne faisant pas partie de l'invention) | **- 55** | **0,4** | **non** |

Exemple 3 : importance du $E_{s(M/V)}$

| Polymère | Tg (°C) | $E_{s(M/V)}$ (μJoules) | Coiffage remodelable |
|---|---|---|---|
| HYSTRETCH V43 | **- 43** | **0,1** | **oui** |
| ECOCRYL XP 4501 (ne faisant pas partie de l'invention) | **- 48** | **900** | **non** |
| CARBOTAC XPD 1811 (ne faisant pas partie de l'invention) | **-43** | **1200** | **non** |

| | |
|---|---|
| AQ 1350 | Polyester (commercialisé par EASTMAN CHEMICAL) |
| HYSTRETCH V-43 | Terpolymère acrylate d'éthyle / acrylamide / acide acrylique (commercialisé par GOODRICH) |
| ECOCRYL XP 4501 | Copolymère acrylique (commercialisé par ATOCHEM) |
| CARBOTAC XPD 1811 | Copolymère acrylique (commercialisé par GOODRICH) |
| KRATON G 1701 | Copolymère en bloc styrène / éthylène propylène (37/63) (commercialisé par SHELL CHIMIE) |
| AMERHOLD DR 25 | Ethylacrylate / méthyl méthacrylate / acide acrylique / acide méthacrylique (commercialisé par AMERCHOL) |
| KRATON G 1701 | Copolymère en bloc styrène / éthylène propylène (37/63) (commercialisé par SHELL CHIMIE) |
| Néocryl BT 67 | Copolymère acrylique / styrène (commercialisé par ZENECA) |

## Revendications

1. Composition de coiffage remodelable, **caractérisée en ce qu'**elle contient, dans un véhicule cosmétique approprié

pour les fibres kératiniques, au moins un polymère choisi de telle sorte que la composition donne après application sur les fibres et séchage, un matériau coiffant ayant une température de transition vitreuse (Tg) inférieure à +10 °C et présentant un profil de décollement défini par au moins :

(a) une force maximale de décollement $F_{max}$ > 1 Newton et
(b) lorsque ladite température Tg est inférieure à -15°C, par une énergie de séparation $E_{s(M/V)}$ du matériau mis en contact avec une surface en verre, inférieure à 300 µJ, et

**en ce qu'**elle se présente sous forme de composition vaporisable, de mousse, de qel ou de lotion.

**2.** Composition selon la revendication 1, **caractérisée en ce que** Fmax est la force maximale de traction, mesurée à l'aide d'un extensomètre, nécessaire pour décoller les surfaces de 38 $mm^2$, respectives de deux supports (A) et (B), rigides, inertes, non-absorbants, placés en regard l'un de l'autre ; lesdites surfaces étant enduites préalablement par ladite composition, à raison de 53/c µg/$mm^2$, c étant la concentration en matière sèche dans la composition (en gramme par gramme de composition) c'est-à-dire le rapport de la masse en matière sèche dans la composition sur la masse totale de la composition, et séchées pendant 24 heures à 22°C sous une humidité relative de 50%, puis soumises pendant 20 secondes à une compression de 3 Newtons et enfin soumises pendant 30 secondes à une traction de vitesse 20 mm/minute.

**3.** Composition selon la revendication 2, **caractérisée en ce que** les supports (A) et (B) sont constitués de polyéthylène, de polypropylène, d'alliage métallique ou de verre.

**4.** Composition selon la revendication 1, **caractérisée en ce que** $E_{s(M/V)}$ est l'énergie fournie par l'extensomètre pour effectuer la séparation des surfaces respectives de 38 $mm^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 2 ou 3 et dont la surface est enduite de la composition, à raison de 53 µg/c /$mm^2$ , c étant la concentration en matière sèche dans la composition (en gramme par gramme de composition), c'est-à-dire le rapport de la masse en matière sèche dans la composition sur la masse totale de la composition,. et séchée pendant 24 heures à 22°C sous une humidité relative de 50% ; les deux surfaces desdits supports (C) et (D) étant soumises ensuite pendant 20 secondes à une compression de 3 Newtons et enfin soumises pendant 30 secondes à une traction de vitesse 20 mm/minute.

**5.** Composition selon la revendication 4, **caractérisée en ce que** $E_{s(M/V)}$ est le travail calculé au moyen de la formule suivante :

$$\int_{Xs1 + 0,05}^{Xs2} F(x)dx$$

où F(x) est la force nécessaire pour produire un déplacement (x) ;
$x_{s1}$ est le déplacement (exprimé en millimètres) produit par la force de traction maximale ;
$X_{S2}$ où le déplacement (exprimé en millimètres) produit par la force de traction permettant la séparation totale des deux surfaces des supports (C) et (D).

**6.** Composition selon l'une quelconque des revendications précédentes, **caractérisée en ce que** le polymère est choisi parmi les polymères fixants anioniques, cationiques, amphotères, non ioniques et leurs mélanges.

**7.** Composition selon l'une quelconque des revendications 1 à 6, **caractérisée en ce que** le ou les polymères sont sous forme solubilisée ou sous forme de dispersion de particules solides de polymère.

**8.** Composition selon la revendication 6, **caractérisée en ce que** les polymères fixants cationiques sont choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

**9.** Composition selon la revendication 6, **caractérisée en ce que** les polymères fixants anioniques sont des polymères comportant des groupements dérivés d'acide carboxylique, sulfonique ou phosphorique et ont un poids moléculaire moyen en poids compris entre environ 500 et 5.000.000.

**10.** Composition selon la revendication 9, **caractérisée en ce que** les polymères fixants anioniques sont des polyesters sulfonés comprenant des unités répétitives pouvant être représentées par la formule générale suivante:

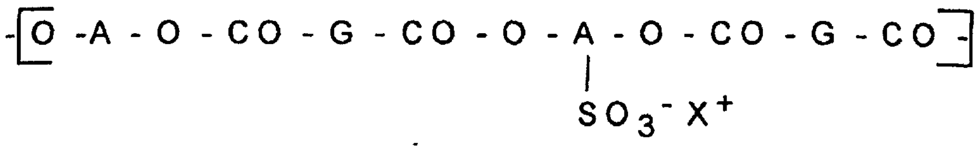

dans laquelle A et G représentent des radicaux divalents et X représente un métal alcalin, en particulier sodium ou potassium.

**11.** Composition selon la revendication 10, **caractérisée en ce que** A représente un radical arylène, en particulier phénylène et G représente un radical alkylène linéaire ou ramifié, éventuellement interrompu par un ou plusieurs atomes d'oxygène, ou cycloalkylène.

**12.** Composition selon la revendication 11, **caractérisée par** en ce que G représente un radical alkylène linéaire ou ramifié, éventuellement interrompu par un ou plusieurs atomes d'oxygène et le radical - O - G - O - est un reste de (poly)alkylène glycol comprenant 1 à 20 unités alkylène glycol; le radical alkylène étant de préférence un radical alkylène inférieur linéaire ou ramifié en $C_2$-$C_4$, et plus préférentiellement un radical éthylène.

**13.** Composition selon la revendication 6, **caractérisée par le fait que** les polymères fixants sont des amphotères choisis parmi les polymères comportant des motifs B et C répartis statistiquement dans la chaîne polymère, où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné; ou bien B et C font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

**14.** Composition selon la revendication 6, **caractérisée en ce que** les polymères fixants non ioniques sont des polyuréthannes.

**15.** Composition selon l'une quelconque des revendications 1 à 14, **caractérisée par le fait que** le ou les polymères sont utilisés seul ou en combinaison avec des additifs cosmétiques usuels tels que des plastifiants ou des neutralisants.

**16.** Composition selon l'une quelconque des revendications 1 à 15, **caractérisée en ce que** le véhicule cosmétiquement acceptable est constitué par un solvant approprié, auquel peuvent être ajoutés des additifs tels que des agents gélifiants ou des agents moussants.

**17.** Composition selon l'une quelconque des revendications 1 à 16, **caractérisée en ce qu'**elle comprend un solvant choisi parmi l'eau, un alcool ou un mélange hydroalcoolique.

**18.** Composition selon l'une quelconque des revendications 1 à 17, **caractérisée en ce qu'**elle comprend en outre une quantité appropriée de propulseur.

**19.** Composition selon la revendication 18, **caractérisée en ce que** le propulseur est constitué par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

**20.** Composition selon l'une quelconque des revendications 1 à 19, **caractérisée par le fait qu'**elle se présente sous forme de produit à rincer tel que des shampooings, des après-shampooings qui, après application sur les fibres kératiniques, rinçage et séchage, conduit à un dépôt en quantité suffisante de matériau coiffant tel que défini dans les revendications précédentes.

**21.** Dispositif aérosol constitué par un récipient contenant une composition aérosol constituée par d'une part une phase liquide (ou jus) contenant au moins un matériau coiffant dans un solvant approprié un propulseur tel que défini dans la revendication 18 ou 19, et un moyen de distribution de ladite composition aérosol.

**22.** Procédé de traitement des fibres kératiniques, en particulier des cheveux, **caractérisé en ce qu'**on applique sur lesdites fibres la composition telle que définie dans les revendications 1 à 20, avant ou après la mise en forme de la coiffure.

**23.** Utilisation d'une composition selon l'une quelconque des revendications 1 à 20 dans une ou pour la fabrication d'une, formulation cosmétique de coiffage remodelable.

**24.** Utilisation d'un polymère tel que défini dans l'une quelconque des revendications 1 à 15 comme matériau coiffant ou pour l'obtention d'un matériau coiffant dans une composition de coiffage remodelable.

## Patentansprüche

**1.** Zusammensetzung, mit der veränderbare Frisuren gestaltet werden können, **dadurch gekennzeichnet, dass** sie in einem für Keratinfasern geeigneten kosmetischen Träger mindestens ein Polymer enthält, das so ausgewählt ist, dass die Zusammensetzung nach dem Auftragen auf die Fasern und dem Trocknen ein Material zur Frisurgestaltung ergibt, das eine Glasübergangstemperatur (Tg) von kleiner +10 °C hat und Ablöseeigenschaften aufweist, die zumindest:

(a) durch eine maximale Ablösekraft $F_{max}$ > 1 Newton und
(b) wenn die Temperatur Tg kleiner -15 °C ist, durch eine Trennungsenergie $E_{S(M/V)}$ des mit einer Glasoberfläche in Kontakt gebrachten Materials von kleiner 300 µJ

definiert sind, und dadurch, dass sie in Form einer zerstäubbaren Zusammensetzung, als Schaum, Gel oder Lotion vorliegt.

**2.** Zusammensetzung nach Anspruch 1, **dadurch gekennzeichnet, dass** $F_{max}$ die maximale mit einem Extensometer bestimmte Zugkraft ist, die erforderlich ist, um die Oberflächen von zwei festen, inerten, nicht absorbierenden Trägern (A) bzw. (B) mit einer Abmessung von 38 $mm^2$, die einander gegenüber angeordnet sind, voneinander zu lösen, wobei die Oberflächen zuvor in einer Menge von 53/c $\mu g/mm^2$ mit der Zusammensetzung beschichtet werden, wobei c die Konzentration der Trockensubstanz in der Zusammensetzung (in Gramm pro Gramm Zusammensetzung), d.h. das Verhältnis der Trockensubstanz in der Zusammensetzung zur Gesamtmasse der Zusammensetzung, bedeutet, anschließend während 24 Stunden bei 22 °C und einer relativen Feuchtigkeit von 50 % getrocknet und dann während 20 Sekunden einer Druckbeanspruchung von 3 Newton sowie schließlich während 30 Sekunden einer Zugbeanspruchung mit einer Geschwindigkeit von 20 mm/Minute unterzogen werden.

**3.** Zusammensetzung nach Anspruch 2, **dadurch gekennzeichnet, dass** die Träger (A) und (B) aus Polyethylen, Polypropylen, einer Metalllegierung oder aus Glas bestehen.

**4.** Zusammensetzung nach Anspruch 1, **dadurch gekennzeichnet, dass** $E_{S(M/V)}$ die Energie ist, die von dem Extensometer für die Trennung der Oberflächen von zwei festen, inerten und nicht absorbierenden Trägern (C) bzw. (D) mit einer Abmessung von 38 $mm^2$, die einander gegenüber angeordnet sind, geliefert wird, wobei einer der Träger aus poliertem Glas besteht und der andere Träger in seiner Art den in Anspruch 2 oder 3 definierten Trägern (A) und (B) entspricht, auf der Oberfläche in einer Menge von 53/c $\mu g/mm^2$ mit der Zusammensetzung beschichtet wird, wobei c die Konzentration der Trockensubstanz in der Zusammensetzung (in Gramm pro Gramm Zusammensetzung), d.h. das Verhältnis der Trockensubstanz in der Zusammensetzung zur Gesamtmasse der Zusammensetzung, bedeutet, und dann während 24 Stunden bei 22 °C und einer relativen Feuchtigkeit von 50 % getrocknet wird, wobei die beiden Oberflächen der Träger (C) und (D) anschließend während 20 Sekunden einer Druckbeanspruchung von 3 Newton sowie schließlich während 30 Sekunden einer Zugbeanspruchung mit einer

Geschwindigkeit von 20 mm/Minute unterzogen werden.

**5.** Zusammensetzung nach Anspruch 4, **dadurch gekennzeichnet, dass** $E_{S(M/V)}$ der nach der folgenden Formel berechneten Arbeit entspricht:

$$\int_{X_{S1}+0,05}^{X_{S2}} F(x)dx$$

worin bedeuten:

- F(x) die Kraft, die erforderlich ist, um eine Verschiebung (x) zu bewirken,
- $x_{S1}$ die (in Millimeter ausgedrückte) Verschiebung, die durch die maximale Zugkraft hervorgerufen wird, und
- $x_{S2}$ die (in Millimeter ausgedrückte) Verschiebung, die durch die Zugkraft hervorgerufen wird, mit der die beiden Oberflächen der Träger (C) und (D) vollständig voneinander getrennt werden können.

**6.** Zusammensetzung nach einem der vorhergehenden Ansprüche, **dadurch gekennzeichnet, dass** das Polymer unter anionischen, kationischen, amphoteren und nichtionischen festigenden Polymeren sowie deren Gemischen ausgewählt ist.

**7.** Zusammensetzung nach einem der Ansprüche 1 bis 6, **dadurch gekennzeichnet, dass** das Polymer oder die Polymere in solubilisierter Form oder in Form einer Dispersion fester Polymerpartikel vorliegen.

**8.** Zusammensetzung nach Anspruch 6, **dadurch gekennzeichnet, dass** die kationischen festigenden Polymere unter Polymeren ausgewählt sind, die in der Polymerkette oder direkt an die Polymerkette gebunden primäre, sekundäre, tertiäre und/oder quartäre Aminogruppen aufweisen und deren Molekulargewicht im Bereich von 500 bis etwa 5 000 000 und vorzugsweise von 1 000 bis 3 000 000 liegt.

**9.** Zusammensetzung nach Anspruch 6, **dadurch gekennzeichnet, dass** es sich bei den anionischen festigenden Polymeren um Polymere mit einem Gewichtsmittel des Molekulargewichts von etwa 500 bis 5 000 000 handelt, die von einer Carbonsäure, Sulfonsäure oder Phosphorsäure abgeleitete Gruppen aufweisen.

**10.** Zusammensetzung nach Anspruch 9, **dadurch gekennzeichnet, dass** die anionischen festigenden Polymere Sulfonsäuregruppen-haltige Polyester mit wiederkehrenden Einheiten sind, die der folgenden allgemeinen Formel entsprechen können:

$$-[O - A - O - CO - G - CO - O - \underset{\underset{SO_3^-X^+}{|}}{A} - O - CO - G - CO -]$$

worin A und G zweiwertige Gruppen und X ein Alkalimetall und insbesondere Natrium oder Kalium bedeuten.

**11.** Zusammensetzung nach Anspruch 10, **dadurch gekennzeichnet, dass** A eine Arylengruppe, insbesondere Phenylen, und G eine geradkettige oder verzweigte, gegebenenfalls mit einem oder mehreren Sauerstoffatomen unterbrochene Alkylengruppe oder eine Cycloalkylengruppe bedeuten.

**12.** Zusammensetzung nach Anspruch 11, **dadurch gekennzeichnet, dass** G eine geradkettige oder verzweigte, gegebenenfalls mit einem oder mehreren Sauerstoffatomen unterbrochene Alkylengruppe bedeutet und die Gruppe - O - G - O - ein (Poly)alkylenglykol mit 1 bis 20 Alkylenglykol-Einheiten ist, wobei die Alkylengruppe vorzugsweise eine geradkettige oder verzweigte niedere Alkylengruppe mit 2 bis 4 Kohlenstoffatomen und noch bevorzugter Ethylen ist.

**13.** Zusammensetzung nach Anspruch 6, **dadurch gekennzeichnet, dass** es sich bei den festigenden Polymeren um amphotere Polymere handelt, die unter Polymeren ausgewählt sind, die in der Polymerkette Einheiten B und C in statistischer Verteilung aufweisen, wobei B eine von einem Monomer mit mindestens einer basischen Gruppe und insbesondere einem basischen Stickstoffatom abgeleitete Einheit bedeutet und C eine von einem sauren Monomer mit einer oder mehreren Carbonsäure-Gruppen oder Sulfonsäure-Gruppen abgeleitete Einheit bezeichnet oder auch B und C Gruppen bedeuten können, die von zwitterionischen Carboxybetain-Monomeren oder Sulfobetain-Monomeren abgeleitet sind; B und C können auch eine kationische Polymerkette mit primären, sekundären, tertiären oder quartären Aminogruppen bedeuten, wobei mindestens eine Aminogruppe eine Carbonsäure-Gruppe oder Sulfonsäure-Gruppe aufweist, die über eine Kohlenwasserstoff-Gruppe gebunden ist, oder B und C liegen in einer Kette eines Polymers mit einer $\alpha,\beta$-Dicarboxyethylen-Einheit vor, wobei eine der Carboxygruppen mit einem Polyamin umgesetzt wurde, das eine oder mehrere primäre oder sekundäre Aminogruppen aufweist.

**14.** Zusammensetzung nach Anspruch 6, **dadurch gekennzeichnet, dass** die nichtionischen festigenden Polymere Polyurethane sind.

**15.** Zusammensetzung nach einem der Ansprüche 1 bis 14, **dadurch gekennzeichnet, dass** das Polymer oder die Polymere allein oder in Kombination mit gebräuchlichen kosmetischen Zusatzstoffen, wie Weichmachern oder Neutralisationsmitteln, verwendet werden.

**16.** Zusammensetzung nach einem der Ansprüche 1 bis 15, **dadurch gekennzeichnet, dass** der kosmetisch akzeptable Träger aus einem geeigneten Lösemittel besteht, zu dem Zusatzstoffe, wie Gelbildner oder Schaumbildner gegeben werden können.

**17.** Zusammensetzung nach einem der Ansprüche 1 bis 16, **dadurch gekennzeichnet, dass** sie ein Lösemittel enthält, das unter Wasser, einem Alkohol oder einem wässerig-alkoholischen Gemisch ausgewählt ist.

**18.** Zusammensetzung nach einem der Ansprüche 1 bis 17, **dadurch gekennzeichnet, dass** sie ferner eine geeignete Menge eines Treibmittels enthält.

**19.** Zusammensetzung nach Anspruch 18, **dadurch gekennzeichnet, dass** das Treibmittel aus gebräuchlichen komprimierten oder verflüssigten Gasen, vorzugsweise Druckluft, komprimiertem Kohlendioxid oder komprimiertem Stickstoff, oder auch aus in der Zusammensetzung löslichen oder nicht löslichen Gasen, wie Dimethylether, fluorierten oder nicht fluorierten Kohlenwasserstoffen und den Gemischen dieser Verbindungen besteht.

**20.** Zusammensetzung nach einem der Ansprüche 1 bis 19, **dadurch gekennzeichnet, dass** sie in Form eines Produkts vorliegt, das ausgespült wird, wie als Haarshampoo oder als Produkt zur Anwendung nach der Haarwäsche, wobei das Produkt nach dem Auftragen auf die Keratinfasern, dem Ausspülen und dem Trocknen zu einer Abscheidung des Materials zur Frisurgestaltung nach einem der vorhergehenden Ansprüche in hinreichender Menge führt.

**21.** Aerosolvorrichtung, die aus einem Behälter, der eine Aerosolzusammensetzung enthält, und einer Vorrichtung zur Verteilung dieser Aerosolzusammensetzung besteht, wobei die Aerosolzusammensetzung zum einen aus einer flüssigen Phase (oder Flüssigkeit), die mindestens ein Material zur Frisurgestaltung in einem geeigneten Lösemittel enthält, und zum anderen aus einem Treibmittel nach Anspruch 18 oder 19 besteht.

**22.** Verfahren zur Behandlung von Keratinfasern und insbesondere der Haare, **dadurch gekennzeichnet, dass** die in den Ansprüchen 1 bis 20 definierte Zusammensetzung vor oder nach der Gestaltung der Frisur auf die Fasern aufgetragen wird.

**23.** Verwendung einer Zusammensetzung nach einem der Ansprüche 1 bis 20 in einer kosmetischen Formulierung zur veränderbaren Frisurgestaltung oder zu deren Herstellung.

**24.** Verwendung eines in einem der Ansprüche 1 bis 15 definierten Polymers als Material zur Frisurgestaltung oder zur Herstellung eines Materials zur Frisurgestaltung in einer Zusammensetzung zur Gestaltung veränderbarer Frisuren.

## Claims

1. Remodellable hair styling composition, **characterized in that** it comprises, in a cosmetic vehicle appropriate for keratinous fibres, at least one polymer selected such that following application to the fibres and drying, the composition gives a styling material having a glass transition temperature (Tg) of less than +10°C and exhibiting a detachment profile defined by at least:

   (a) a maximum detachment force $F_{max}$ > 1 newton and
   (b) when the said temperature Tg is lower than -15°C, by a separation energy $E_{s(m/g)}$ of the material placed in contact with a glass surface of less than 300 μJ, and

   **in that** it is provided in the form of a vaporizable composition, mousse, gel or lotion.

2. Composition according to Claim 1, **characterized in that** Fmax is the maximum tensile force, measured using an extensometer, required to detach the respective 38 $mm^2$ surfaces of two rigid, inert and non-absorbent substrates (A) and (B) placed opposite one another, the said surfaces being coated beforehand with the said composition at a rate of 53/c $\mu g/mm^2$, c being the dry matter concentration in the composition (in grams per gram of composition), i.e. the ratio of the mass of dry matter in the composition to the total mass of the composition, and the said surfaces being dried for 24 hours at 22°C and a relative humidity of 50%, then subjected for 20 seconds to a compressive force of 3 newtons and finally subjected for 30 seconds to tension at a rate of 20 mm/minute.

3. Composition according to Claim 2, **characterized in that** the substrates (A) and (B) consist of polyethylene, polypropylene, metal alloy or glass.

4. Composition according to Claim 1, **characterized in that** $E_{s(m/g)}$ is the energy supplied by the extensometer in order to bring about the separation of the respective 38 $mm^2$ surfaces of two rigid, inert and non-absorbent substrates (C) and (D) placed opposite one another; one of the said substrates consisting of polished glass and the other of the said substrates being identical in nature to the substrates (A) and (B) as defined in Claim 2 or 3 and having a surface which is coated with the composition at a rate of 53 $\mu g/c\ /mm^2$, c being the dry matter concentration in the composition (in grams per gram of composition), i.e. the ratio of the mass of dry matter in the composition to the total mass of the composition, and dried for 24 hours at 22°C and a relative humidity of 50%; the two surfaces of the said substrates (C) and (D) being subjected subsequently for 20 seconds to a compressive force of 3 newtons and finally subjected for 30 seconds to tension at a rate of 20 mm/minute.

5. Composition according to Claim 4, **characterized in that** $E_{s(m/g)}$ is the work calculated using the following formula:

$$\int_{X_{s1}+0.05}^{X_{s2}} F(x)dx$$

   where F(x) is the force required to produce a displacement (x);
   $x_{s1}$ is the displacement (in millimetres) produced by the maximum tensile force;
   $x_{s2}$ where the displacement (in millimetres) produced by the tensile force which permits the total separation of the two surfaces of the substrates (C) and (D).

6. Composition according to any one of the preceding claims, **characterized in that** the polymer is selected from anionic, cationic, amphoteric and nonionic fixative polymers and mixtures thereof.

7. Composition according to any one of Claims 1 to 6, **characterized in that** the polymer or polymers are in solubilized form or in the form of dispersion of solid polymer particles.

8. Composition according to Claim 6, **characterized in that** the cationic fixative polymers are selected from polymers containing primary, secondary, tertiary and/or quaternary amine groups forming part of the polymer chain or linked directly to it and having a molecular weight of between 500 and approximately 5,000,000 and preferably between

1000 and 3,000,000.

**9.** Composition according to Claim 6, **characterized in that** the anionic fixative polymers are polymers containing groups derived from carboxylic, sulphonic or phosphoric acid and have a weight-average molecular weight of between approximately 500 and 5,000,000.

**10.** Composition according to Claim 9, **characterized in that** the anionic fixative polymers are sulphonated polyesters comprising repeating units representable by the following general formula:

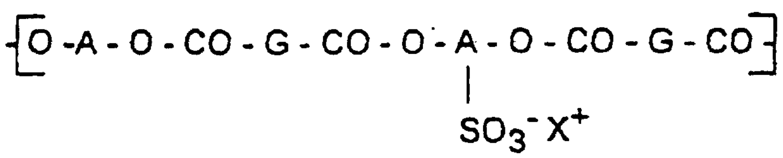

in which A and G represent divalent radicals and X represents an alkali metal, especially sodium or potassium.

**11.** Composition according to Claim 10, **characterized in that** A represents an arylene radical, especially phenylene, and G represents a linear or branched alkylene radical optionally interrupted by one or more oxygen atoms, or a cycloalkylene radical.

**12.** Composition according to Claim 11, **characterized in that** G represents a linear or branched alkylene radical optionally interrupted by one or more oxygen atoms and the radical - O - G - O - is a (poly)alkylene glycol residue containing 1 to 20 alkylene glycol units; the alkylene radical being preferably a lower, linear or branched $C_2$-$C_4$ alkylene radical and, more preferably, an ethylene radical.

**13.** Composition according to Claim 6, **characterized in that** the fixative polymers are amphoterics selected from polymers comprising units B and C distributed randomly in the polymer chain, where B denotes a unit deriving from a monomer containing at least one basic function, in particular a basic nitrogen atom, and C denotes a unit deriving from an acidic monomer containing one or more carboxyl or sulpho groups, or else B and C can denote groups deriving from zwitterionic carboxybetaine or sulphobetaine monomers; B and C may also denote a cationic polymer chain containing primary, secondary, tertiary or quaternary amine groups, in which at least one of the amine groups carries a carboxyl or sulpho group linked by way of a hydrocarbon radical; or else B and C form part of a chain of a polymer having an $\alpha,\beta$-dicarboxy ethylene unit in which one of the carboxyl groups has been reacted with a polyamine containing one or more primary or secondary amine groups.

**14.** Composition according to Claim 6, **characterized in that** the nonionic fixative polymers are polyurethanes.

**15.** Composition according to any one of Claims 1 to 14, **characterized in that** the polymer or polymers are employed alone or in combination with conventional cosmetic additives such as plasticizers or neutralizing agents.

**16.** Composition according to any one of Claims 1 to 15, **characterized in that** the cosmetically acceptable vehicle consists of an appropriate solvent to which may be added additives such as gelling agents or foaming agents.

**17.** Composition according to any one of Claims 1 to 16, **characterized in that** it comprises a solvent selected from water, an alcohol or an aqueous-alcoholic mixture.

**18.** Composition according to any one of Claims 1 to 17, **characterized in that** it additionally comprises an appropriate amount of propellant.

**19.** Composition according to Claim 18, **characterized in that** the propellant consists of the conventional liquefied or compressed gases, preferably compressed air, carbon dioxide or nitrogen, or else a gas which is soluble or otherwise in the composition, such as dimethyl ether, fluorinated or non-fluorinated hydrocarbons, and mixtures thereof.

**20.** Composition according to any one of Claims 1 to 19, **characterized in that** it is provided in the form of a rinsable

product such as shampoos, conditioners, which, following application to the keratinous fibres, rinsing and drying, leads to a deposit, in an adequate amount, of styling material as defined in the preceding claims.

**21.** Aerosol device consisting of a vessel containing an aerosol composition consisting on the one hand of a liquid phase (or juice) containing at least one styling material in an appropriate solvent, a propellant as defined in Claim 18 or 19, and a means of dispensing the said aerosol composition.

**22.** Method of treating keratinous fibres, especially hair, **characterized in that** the composition as defined in Claims 1 to 20 is applied to the said fibres before or after the shaping of the hairstyle.

**23.** Use of a composition according to any one of Claims 1 to 20 in, or for the preparation of, a cosmetic remodellable hair styling formulation.

**24.** Use of a polymer as defined in any one of Claims 1 to 15 as a styling material, or for producing a styling material, in a remodellable hair styling composition.